# EUROPÄISCHE PATENTSCHRIFT

(11) **EP 3 863 520 B1**
(45) Veröffentlichungstag und Bekanntmachung des Hinweises auf die Patenterteilung: **30.08.2023**
(21) Anmeldenummer: 19795460.5
(22) Anmeldetag: 09.10.2019
(51) Int. Cl.: A61B 8/00, A61B 8/08, A61G 7/075

(54) **HALTEVORRICHTUNG FÜR EINE ULTRASCHALLSONDE UND PERSONENAUFNAHME MIT EINER HALTEVORRICHTUNG**
FIXTURE FOR AN ULTRASOUND PROBE AND RECEPTACLE FOR A PERSON HAVING A FIXTURE
DISPOSITIF DE RETENUE POUR UNE SONDE À ULTRASONS ET ÉLÉMENT DE RÉCEPTION DE PERSONNE COMPRENANT UN DISPOSITIF DE RETENUE

(30) Priorität: 11.10.2018 DE 102018125155; 04.10.2019 DE 202019105479 U
(43) Veröffentlichungstag der Anmeldung: 18.08.2021
(73) Patentinhaber: Sono-Mount GmbH, 63303 Dreieich (DE)
(72) Erfinder: SCHÜTZ, Michael, 63303 Dreieich (DE)
(74) Vertreter: Verscht, Thomas Kurt Albert
(86) Internationale Anmeldenummer: PCT/EP2019/077421
(87) Internationale Veröffentlichungsnummer: WO 2020/074615

(56) Entgegenhaltungen:
- US-A1- 2014 213 904
- US-A1- 2016 184 126
- US-A1- 2018 263 596
- J MAGUNIA ET AL: "Blockade of the distal sciatic nerve with the patient in the supine position using a newly developed position aid with integrated ultrasound probe holder", INTERNATIONAL JOURNAL OF CLINICAL ANESTHESIA AND RESEARCH, Bd. 3, Nr. 1, 9. Juli 2019 (2019-07-09), Seiten 003-006, XP055658382, DOI: 10.29328/journal.ijcar.1001012

## Beschreibung

Die vorliegende Erfindung betrifft das technische Gebiet der Medizintechnik. Insbesondere betrifft die vorliegende Erfindung eine Haltevorrichtung für eine Ultraschallsonde und eine Personenaufnahme.

In der Medizintechnik werden Ultraschalluntersuchungen unter Verwendung von Ultraschallsonden durchgeführt. Hierbei können tief liegenden Strukturen in menschlichen oder tierischen Geweben (Blutgefäße, Nerven, Sehnen, Muskeln, Tumoren, Lymphknoten usw.) sichtbar gemacht werden. Eine Ultraschalluntersuchung wird üblicherweise von einem Arzt oder einer medizinischen Fachkraft durchgeführt, die eine Ultraschallsonde an die zu untersuchende Stelle führt und das tieferliegende Gewebe oder Organe analysiert. Eine Untersuchung wird bisher üblicherweise mit einer Ultraschallsonde durchgeführt, die über ein Versorgungskabel an ein Ultraschallgerät angeschlossenen ist. Die Ultraschallsonde ist Teil eines Ultraschallgerätes, mit dem üblicherweise zweidimensionale Aufnahmen in Form von Bildern oder Filmen des zu untersuchenden Gewebes wiedergegeben und festgehalten werden. Die Aufnahmen geschehen vorteilhafterweise in Echtzeit.

US 2014/213904 A1 beschreibt ein System zum Überwachen von Bewegungen von Patienten während einer Behandlung von Krebs mit einem Strahlentherapiesystem. Hierbei weist das System eine Strahlungseinheit und ein Patientenpositionierungssystem auf. Bei einer Anwendung ist eine Platzierung eines Ultraschallwandlers nahe einem Halswirbel eines Patienten vorgesehen, um Bewegungen des Patienten während einer Tumorbehandlung im Halsbereich zu erkennen. Der Ultraschallwandler ist in einer Nackenstützstruktur des Patientenpositionierungssystem integriert angeordnet zur Stützung des Kopfes des Patienten während der Behandlung. Der Ultraschallwandler erzeugt Ultraschallwellen, die von dem Halswirbel reflektiert werden. Anhand der Zeitabstände zwischen den erzeugten Signalen und den entsprechenden reflektierten Signalen ist es möglich, Bewegungen des Patienten während der Strahlenbehandlung zu erkennen.

US 2018/263596 A1 beschreibt einen Sondenadapter, der eine Ultraschallsonde in einem Winkel in Bezug auf ein abzubildendes Objekt hält, wie einem Körperteil eines menschlichen Körpers. Eine Kontaktfläche des Sondenadapters weist eine Form auf, beispielsweise eine Krümmung, die sich entlang eines Außenumfangs eines Querschnitts des abzubildenden Objekts erstreckt, beispielsweise eine gekrümmte Oberfläche eines Oberschenkels. Auf diese Weise wird eine große Kontaktfläche zwischen der Sonde und dem abzubildenden Objekt durch den Sondenadapter gehalten, auch während die Sonde entlang der Oberfläche des abzubildenden Objekts bewegt wird.

US 2016/184126 A1 beschreibt eine medizinische Vorrichtung zur Behandlung und Ruhigstellung einer Gliedmaße eines Patienten unter Verwendung einer Kombination aus einer Schiene und einem Kissen. Hierbei kann auch eine Ultraschallsonde verwendet werden, die in einer Aussparung angeordnet ist und Zugang zu der Unterseite der Schiene ermöglicht.

Ultraschallsonden werden nicht nur zur Analyse und Diagnose an Geweben und Organen verwendet, sondern auch im Zusammenhang mit Punktionen, um einen medizinischen Eingriff vorzubereiten, wie beispielsweise eine Operation. Bei solchen Punktionen werden mit Hilfe von Punktionsnadeln Medikamente verabreicht. Hierbei wird bei einer Punktion ein Lokalanästhetikum in die Nähe einer oder mehrerer vorbestimmter Nervenbahnen gespritzt. Diese lokale Verabreichung des Mittels hat den Vorteil, dass nur bestimmte Bereiche oder bestimmte Nervenbahnen betäubt werden, um beispielsweise eine Vollnarkose zu vermeiden oder eine Schmerztherapie durchzuführen.

Zur Verabreichung des Lokalanästhetikums werden Punktionsnadeln verwendet, um das Lokalanästhetikum gezielt an die gewünschte Stelle über die Punktionsnadel zu verabreichen. Es ist bei solchen Punktionen wünschenswert, die Punktion in Echtzeit zu überwachen. Hierzu wird die Punktionsnadel über ein Echtzeit-Ultraschallbild sichtbar gemacht, während sich die Punktionsnadel der gewünschten Stelle des Organs oder Nerven innerhalb des Körpers des Patienten nähert.

Bei Punktionen ist es bisher nachteilig, dass der Arzt oder das medizinische Personal mehrere Untersuchungsschritte zeitgleich durchführen muss. Zunächst wird beispielsweise die Ultraschallsonde mit einer Hand gehalten und auf das zu suchende Areal bzw. Gewebe gepresst. Mit der zweiten Hand des Untersuchers wird eine Punktionsnadel zur angezielten Struktur, insbesondere zu tiefliegendem Gewebe bzw. Nervenbahnen geführt. Um die angezielte Struktur zu erreichen, weist die Punktionsnadel eine ausreichende Länge aus und wird unterhalb der Hautoberfläche im Inneren des Körpers mit einer Ultraschallsonde sichtbar gemacht. Mit Hilfe von Ultraschallwellen, die in das Gewebe eindringen, können die Bewegungen der Nadel im Gewebe sichtbar gemacht werden. Die Bewegungen der Nadel in Echtzeit können als zweidimensionaler Film auf einem Bildschirm abgebildet werden. Auf diese Weise ist es möglich, dass die zweite Hand des Untersuchers die Punktionsnadel an die gewünschte Struktur im Innern des Gewebes zielgenau heranführen kann. Der Untersucher hält somit mit einer Hand die Ultraschallsonde, mit der anderen Hand die Punktionsnadel und überwacht gleichzeitig auf einem Bildschirm seine Führung der Punktionsnadel in der Gewebestruktur.

Aufgrund der erforderlichen Verwendung beider Hände des Untersuchers, die koordiniert werden müssen, ist dieses Verfahren einer Punktion mit Ultraschalleinsatz sehr komplex und schwer zu erlernen. Es wurden daher Vorrichtungen zum Punktieren entwickelt, die Einstechhilfen vorsehen, um eine Handhabung bei der Aufnahme von Ultraschall-Echolotbildern zu vereinfachen. Eine solche Vorrichtung wird beispielsweise in DE 29 36 259 A1 beschrieben.

In bestimmten Bereichen des menschlichen Körpers wie zum Beispiel auf der Körperrückseite, wie Oberschenkel oder Oberarm kommen zusätzliche Schwierigkeiten hinzu, da Körperrückseiten für das Aufsetzen der Ultraschallsonde kaum zugänglich sind. Es bleibt kaum Raum für die Ultraschallsonde unterhalb des zu untersuchenden Körperteils aufgrund der Abmessungen der Ultraschallsonde. Derzeit hilft man sich damit, dass für eine solche Untersuchung ein zu untersuchender Körperteil eines Patienten, insbesondere eine zu untersuchende Extremität, selbst in eine geeignete Lage gebracht wird. Dies kann bedeuten, dass Verlagerungen des Patienten notwendig werden, um geeignete Untersuchungsbedingungen schaffen zu können. Der Patient muss hierfür beispielsweise in eine Seitenlagerung oder Bauchlagerung vor der eigentlichen medizinischen Untersuchung gebracht werden. Dies hat jedoch den Nachteil, dass bestimmte Untersuchungsbedingungen bei verletzten, alten oder gebrechlichen Patienten schwer oder gar nicht umsetzbar sind. Die Folge kann eine notwendige Vollnarkose sein, da notwendige Bereiche zur lokalen Betäubung nicht zugänglich sind.

Es ist daher wünschenswert bei der Verwendung von Ultraschallsonden Untersuchungsbedingungen zu schaffen, bei denen ein Patient möglichst wenig verlagert werden muss. Eine geringe Verlagerung ist insbesondere bei der Untersuchung von Körperrückseiten des Patienten wünschenswert. Unter Körperrückseiten sind in diesem Zusammenhang die Körperpartien des Patienten zu verstehen, die dem untersuchenden Personal nicht direkt zugänglich sind. Die Unzugänglichkeit kann dadurch begründet sein, dass der Patient auf dem entsprechenden Körperteil liegt bzw. dieses durch seine Körperhaltung verdeckt.

Es besteht somit ein Bedarf Untersuchungsmöglichkeiten bei einer Ultraschalluntersuchung an einem Patienten zu verbessern.

Diese Aufgabe wird mit einer Haltevorrichtung nach Anspruch 1 gelöst. Ferner wird diese Aufgabe mit einer Personenaufnahme gemäß Anspruch 14 gelöst. Weitere bevorzugte Ausführungsformen der Erfindung sind in den abhängigen Patentansprüchen offenbart.

Es wird eine Haltevorrichtung für eine Ultraschallsonde vorgeschlagen, aufweisend einen Grundkörper mit einer Grundfläche. Hierbei weist der Grundkörper eine Aufnahmeöffnung auf zur Aufnahme einer Ultraschallsonde. Ferner ist vorgesehen, dass die Haltevorrichtung eine Auflagefläche aufweist zur Stützung eines zu untersuchenden Körperteils. Hierbei ist die Aufnahmeöffnung in der Auflagefläche angeordnet ist, so dass mit einer aufgenommenen Ultraschallsonde in der Aufnahmeöffnung der zu untersuchende Körperteil aus der Auflagefläche heraus beschallbar ist. Die vorgeschlagene Haltevorrichtung nimmt zumindest teilweise oder vorzugsweise vollständig in deren Grundkörper eine Ultraschallsonde auf und kann zu einer bildgebenden Darstellung eines zu untersuchenden Körperteils einer Person verwendet werden.

Ferner ist bei der erfindungsgemäßen Haltevorrichtung vorgesehen, dass der zu untersuchende Körperteil ein Oberschenkel einer Person ist. Insbesondere kann mit Vorteil die Rückseite eines Oberschenkels untersucht werden. Beispielsweise kann an der Rückseite eines Oberschenkels mit Hilfe der vorgeschlagenen Haltevorrichtung ein Nerv innerhalb des Oberschenkels punktiert werden. Dies ist möglich, da die Haltevorrichtung als Auflagefläche für den Oberschenkel dienen kann.

In einer weiteren Ausgestaltung der Haltevorrichtung kann vorgesehen sein, dass die Kontur der Öffnung der Kontur der Ultraschallsonde derart angepasst ist. Auf diese Weise ist die Ultraschallsonde relativ zum Körperteil einer Person innerhalb der Haltevorrichtung fest fixiert.

In einer weiteren Ausgestaltung kann die Haltevorrichtung so ausgestaltet sein, dass die Verkabelung der Ultraschallsonde in dafür vorgesehenen, in der Patientenunterlage integrierten Kanälen vorgesehen ist.

Die erfindungsgemäße Haltevorrichtung wird im bestimmungsgemäßen Gebrauch während der Untersuchung eines Patienten verwendet. Die Ultraschallsonde wird hierbei innerhalb des Grundkörpers aufgenommen, während ein Patient an der Haltevorrichtung platziert wird und ein Körperteil mit Ultraschall untersucht wird. Hierbei wird die Ultraschallsonde, auch Ultraschallkopf genannt, vollständig oder zumindest teilweise in dem Grundkörper aufgenommen. Zur Aufnahme der Ultraschallsonde wird eine Aussparung in dem Grundkörper vorgesehen. Erfindungsgemäß wird die Auflagekraft bzw. die Schwerkraft der Person bzw. des zu untersuchenden Körperteils genutzt, um die Ultraschallsonde innerhalb ihrer Halterung bzw. der Aussparung in Bezug auf das darüber liegende Gewebe in einer fixierten Position zu halten.

Der Grundkörper hat vorzugsweise eine Höhe von etwa 5 cm bis etwa 20 cm, wobei die Mindesthöhe von der Geometrie der Ultraschallsonde abhängt. Vorzugsweise hat der Grundköper eine Breite von etwa 15 cm bis 45 cm. Ferner ist vorgesehen, dass die Haltevorrichtung ein Gewicht im Bereich von etwa 100 Gramm bis etwa 2,5 Kilogramm aufweist. Durch eine entsprechende Formgebung der Stützfläche wird das Gewicht des zu untersuchenden Körperteils gleichmäßig verteilt und gleichzeitig stabilisiert, so dass der Körperteil während der Ultraschalluntersuchung ruhig auf der Auflagefläche der Haltevorrichtung liegt.

In einer Ausführungsform der Haltevorrichtung kann vorgesehen werden, dass der Grundkörper einstückig ausgebildet ist.

Ein einstückiger Grundkörper ist einfach herzustellen und auch einfach zu handhaben, beispielsweise zu desinfizieren bzw. zu sterilisieren. In bevorzugten Ausführungsformen kann auch die gesamte Haltevorrichtung einstückig ausgebildet sein, insbesondere wenn diese ohne schwenkbare Einrichtungen ausgeführt ist.

Ein einstückiger Grundkörper kann beispielsweise gespritzt, gedruckt, gepresst oder gegossen hergestellt werden. Hierbei können beispielsweise Vergussmassen in einem Spritzgießverfahren verwendet werden. Ein gedruckter Grundkörper kann beispielsweise in einem 3D-Druckverfahren hergestellt werden. Das gedruckte Material kann beispielsweise Kunststoff sein. Ein gepresster Grundkörper kann beispielsweise aus Styroporteilen, wie Styproporkugeln, hergestellt werden, die lose in eine Form eingeführt werden, um darin durch mechanischen Druck aneinander gepresst zu werden. Ein gegossener Grundkörper kann beispielsweise aus Kunststoffmasse, Gummimasse oder Metallmaterial hergestellt werden.

Vorteilhafterweise kann vorgesehen werden, dass der Grundkörper deformierbar ist.

Ein deformierbarer Grundkörper kann beispielsweise aus gummiartigen Material, Silikonmaterial oder aus Schaumstoffmaterial hergestellt sein. Anstelle den gesamten Grundkörper aus einem deformierbaren Material herzustellen, kann alternativ der Grundkörper eine deformierbare Beschichtung aufweisen, die sich beispielsweise über seine Außenfläche oder zumindest teilweise über seine Außenfläche erstreckt. Die Eigenschaft deformierbar bedeutet, dass der Grundkörper bzw. seine Beschichtung die ursprüngliche geometrische Form verändern kann aufgrund einer einwirkenden Schwerkraft eines zu untersuchenden Körperteils des Patienten während einer Untersuchung mit einer Ultraschallsonde. Der Grundkörper ist durch seine deformierbare Eigenschaft elastisch ausgebildet und verformt sich reversibel unter dem Gewicht eines aufgelegten Körperteils auf die Haltevorrichtung.

Ferner kann mit Vorteil vorgesehen werden, dass die Grundfläche der Haltevorrichtung im bestimmungsgemäßen Gebrauch durch eine Deformation des Grundkörpers gebildet wird.

Ein deformierbarer Grundkörper bzw. eine deformierbare Außenfläche des Grundkörpers hat den Vorteil, dass im bestimmungsgemäßen Gebrauch eine Grundfläche am Grundkörper vorhanden ist, die durch das Gewicht des zu untersuchenden Körperteils hervorgerufen wird.

Dies ermöglicht einfache geometrische Formen für den Grundkörper zu wählen, beispielsweise einen zylindrischer Grundkörper, der sich im bestimmungsgemäßen Gebrauch deformiert und von der zylindrischen Grundform abweicht. Auf diese Weise ist es möglich, alleine durch die Schwerkraft bzw. das Gewicht des Körperteils den Grundkörper aufgrund seiner Deformation von einem Wegrollen zu verhindern.

Die Deformation ist hierbei mit Vorteil reversibel, d.h. nach einer Untersuchung des Patienten nimmt der Grundkörper seine ursprüngliche Form wieder an. Die Deformation liegt vorzugsweise im Millimeterbereich.

Hat beispielsweise der Grundkörper die Form eines Zylinders, so wird die Grundfläche durch Deformation der Mantelfläche des Zylinders gebildet. Auf diese Weise bildet der zuvor zylindrische Körper eine Grundfläche für die Dauer der Untersuchung und wird vom Wegrollen gehindert. Die zylindrische Form vor der Untersuchung und während der Positionierung des Patienten hat den Vorteil, dass zu diesem Zeitpunkt wenig oder keine Belastung auf dem Grundkörper verübt wird, so dass dieser abrollen kann und während des Positioniervorgangs leicht in seine gewünschte Position bewegt werden kann.

Insgesamt wird auf diese Weise eine Haltevorrichtung bereitgestellt, die auf einfache Weise hergestellt werden kann und vielfältig für Untersuchungen an Patienten eingesetzt werden kann.

In einer Ausführungsform der Haltevorrichtung kann vorgesehen werden, dass die Haltevorrichtung eine Kabelführung aufweist.

Hierbei kann vorteilhafterweise ein Kabel der Ultraschallsonde von der Kabelführung aufgenommen werden, so dass das Kabel im Inneren des Grundkörpers geführt wird. Dies hat den Vorteil, dass das Kabel der Ultraschallsonde während einer Untersuchung nicht weiter stört. Die Kabelführung kann beispielsweise als Hohlraum im Grundkörper ausgeführt sein, insbesondere als ein Kanal, der zumindest teilweise im Inneren des Grundkörpers verläuft. Sofern die Ultraschallsonde kabellos ausgeführt ist, wird keine Kabelführung notwendig sein, so dass der Kabelkanal entfallen kann.

Ferner weist die Haltevorrichtung vorteilhafterweise eine Zielhilfe auf.

Eine Zielhilfe wird vorzugsweise für den Fall verwendet, dass zusätzlich zu einer Ultraschalluntersuchung eine Injektion oder eine Punktion vorgenommen werden soll. Mit der Zielhilfe kann ein Arzt oder das medizinisches Personal auf einfache Weise eine geeignete Einstichstelle für eine Hohlnadel auffinden und gezielt an eine gewünschte Stelle im Körper des Patienten führen. Somit dient die Zielhilfe als Orientierung und Richtungsgebung für das Führen der Hohlnadel an die Einstichstelle und innerhalb des Körpers des Patienten.

Da die Haltevorrichtung im bestimmungsgemäßen Gebrauch unterhalb eines Körperteils eines Patienten positioniert ist, kann es vorkommen, dass je nach Größe des Körperteils die Haltevorrichtung vollständig durch den aufliegenden Körperteil bedeckt wird. Die Zielhilfe kann daher seitlich am Grundkörper angeordnet sein, einseitig oder beidseitig, da die Seitenflächen bzw. zumindest eine der Seitenflächen im bestimmungsgemäßen Gebrauch zugänglich sind.

Mit Vorteil kann vorgesehen werden, dass die Zielhilfe eine visuelle Markierung ist.

Eine visuelle Markierung kann beispielsweise als Pfeil oder Linie ausgeführt sein, um eine Hohlnadel an einer gewünschten Stelle am Patienten zu positionieren.

Ferner kann mit Vorteil vorgesehen werden, dass die Zielhilfe ein tastbarer Vorsprung ist.

Es kann vorkommen, dass je nach Lage und Körperteil der Person die Haltevorrichtung vollständig oder teilweise verdeckt ist. Mit einem tastbaren Vorsprung oder einer Vielzahl tastbaren Vorsprüngen kann dennoch eine Hohlnadel präzise positioniert an der Einstichstelle positioniert werden. Ferner kann die Zielhilfe während der Behandlung des Patienten verwendet werden, um die Hohlnadel anhand eines Echtzeit-Ultraschall-Filmes im inneren des Körpers des Patienten zu bewegen.

In einer weitere Ausführungsform der Haltevorrichtung kann vorgesehen werden, dass die Zielhilfe eine Vertiefung ist.

Eine Vertiefung als Zielhilfe hat den Vorteil, dass kein weiterer Raum für die Zielhilfe in Anspruch genommen wird und der Patient keine Unebenheit bei der Auflage des zu untersuchenden Körperteils spürt.

In einer weiteren Ausführungsform kann die Haltevorrichtung eine oder eine Vielzahl von Tasthilfen aufweisen. Eine solche Tasthilfe kann an einer Seitenwand des Grundkörpers der Haltevorrichtung angeordnet sein und zur verbesserten Führung einer Injektionsnadel verwendet werden, indem das ärztliche Personal entlang der Zielhilfe mit ihren Fingern tastet, um eine geeignete Einstichstelle am Körperteil des Patienten zu finden. Die Tasthilfe kann als hervorspringende Geometrie an dem Grundkörper vorhanden sein.

Mit Vorteil kann eine schwenkbare Einrichtung zur Aufnahme und Schwenkung der Ultraschallsonde vorgesehen sein.

Mit einer schwenkbaren Einrichtung kann die Ultraschallsonde beweglich gelagert werden, so dass ein vorbestimmter Winkelbereich als Sichtfenster der Ultraschallsonde abgedeckt werden kann. Mit einer schwenkbaren Einrichtung, die die Ultraschallsonde aufnimmt, kann auf einfache Weise die Sichtrichtung der Ultraschallsonde verändert werden, ohne dass der Patient hierzu bewegt werden muss.

Ferner kann vorteilhafterweise vorgesehen sein, dass die schwenkbare Einrichtung eine drehbar gelagerte Walze aufweist und die Walze mindestens eine Öffnung zur Aufnahme der Ultraschallsonde aufweist.

Die Walze hat somit die Funktionen einer Verlagerung der Ultraschallsonde und gleichzeitig die Funktion einer Halterung der Ultraschallsonde. Die Walze kann beispielsweise mit Kugellager versehen sein, so dass wenig Kraft zur Verlagerung benötigt wird.

In einer weiteren Ausführungsform kann die Haltevorrichtung einen Griff aufweisen.

Der Grundkörper kann beispielsweise einen Griff aufweisen, der beispielsweise als Bügelgriff geformt ist. Mit dem Griff ist es möglich, die Haltevorrichtung am Patienten auf einfache Weise zu positionieren. Ferner kann der Griff auch als Transporthilfe der Haltevorrichtung verwendet werden. Der Griff kann an einer oder beiden Seitenflächen des Grundkörpers angeordnet sein. Ferner kann der Griff auch an der Längsseite der Stützfläche angeordnet sein.

Ferner ist von Vorteil, dass die Ultraschallsonde vollständig in dem Grundkörper aufnehmbar ist.

Bei vollständiger Aufnahme der Ultraschallsonde in den Grundkörper entsteht eine glatte Oberfläche am Grundkörper, die eine Auflage eines Körperteils eines Patienten ermöglicht. Die glatte Oberfläche hat den Vorteil, dass eine gute Ankopplung zwischen Ultraschallsonde und Körperteil gewährleistet werden kann, ohne dass Lufteinschlüsse im Sichtbereich der Ultraschallsonde entstehen. Dies verbessert die Aufnahmequalität der Ultraschallsonde. Ferner ist es möglich, dass der Ultraschallkopf zusätzlich mit einem Kontaktgel versehen wird, das zusätzlich die Ultraschallaufnahmen verbessert.

In einer Ausführungsform der Haltvorrichtung kann vorgesehen werden, dass die im Grundkörper aufgenommene Ultraschallsonde und die Auflagefläche eine gemeinsame stufenfreie Fläche bilden. Diese Fläche hat keine Stufe, d.h. sie ist absatzlos. Ferner kann die Auflagefläche auch gekrümmt verlaufen, beispielsweise als Teil eines Zylindermantels.

In einer weiteren Ausführungsform der Haltevorrichtung kann der Grundkörper eine rechteckige Grundfläche aufweisen. Die rechteckige Grundfläche dient als Standfläche des Grundkörpers. Sie kann beispielsweise etwa 200 bis etwa 900 Quadratzentmeter betragen.

In einer weiteren Ausführungsform kann die Ausstrahlung von Schallwellen aus der Ultraschallsonde entgegen der Schwerkraftrichtung gerichtet ist. Auf diese Weise kann durch die Schwerkraft der zu untersuchende Körperteil auf die Auflagefläche drücken und dort gleichzeitig Schallwellen entgegen der Schwerkraft austreten.

In einer weiteren Ausführungsform kann der Grundköper achsensymmetrisch sein. Dies hat den Vorteil, dass für ein linkes Bein wie auch für ein rechtes Bein die gleiche Haltevorrichtung verwendet werden kann. Die Halterichtung hat in Querrichtung, d.h. bei der Anwendung in Bezug auf die rechte oder linke Seitenbegrenzung der Haltevorrichtung, keine Vorzugsrichtung.

In einem weiteren Ausführungsbeispiel kann vorgesehen werden, dass im bestimmungsgemäßen Gebrauch der Haltevorrichtung mit Hilfe der Schwerkraft der zu untersuchende Körperteil auf die Auflagefläche gedrückt wird. Es wird demnach der zu untersuchende Körperteil verwendet, um mit dessen Eigengewicht auf den Schallkopf zu drücken. Damit die Schallwellen problemlos aus der Schallkopfoberfläche in den Oberschenkel eindringen können, wird ein Ankopplungsmedium zwischen Schallkopf und Oberschenkel verwendet, z.B. in Form einer wasserhaltige Flüssigkeit oder eines Gels. Auf einfache Weise mit Hilfe der Schwerkraftwirkung wird ein luftfreier Kontakt zwischen Schallkopfoberfläche und Körperteil hergestellt. Insgesamt verbessert dies die Qualität der Ultraschallaufnahme. Hierbei kann eine zeitlich kontinuierliche Serie von Aufnahmen aufgenommen werden oder ein einzelnes Standbild. Eine zeitlich kontinuierliche Aufnahme in Echtzeit ist besonders von Vorteil, um eine Punktion im Inneren des untersuchten Körperteils durchzuführen.

In einer weiteren Ausgestaltung kann vorgesehen sein, dass die Ultraschallsonde an dem Grundkörper übersteht.

Bei dieser Variante ist im bestimmungsgemäßen Gebrauch erwünscht, dass die Ultraschallsonde nicht flächig mit dem Grundkörper abschließt, sondern aus diesem teilweise hervortritt. Das Hervortreten beschränkt sich auf wenige Millimeter, beispielsweise im Bereich von etwa 2 bis etwa 5 Millimeter, vorzugsweise im Bereich von etwa 3 bis etwa 4 Millimeter. Dieser Vorsprung an dem Grundkörper im bestimmungsgemäßen Gebrauch hat den Vorteil, dass eine Spannung der Haut des Patienten erfolgen kann, so dass ein auf der Ultraschallsonde liegender Körperteil gezielt geformt wird bzw. die Haut des Patienten gespannt wird. Diese Verformung verbessert die Kontaktfläche zwischen dem zu untersuchenden Körperbereich des Patienten und der Ultraschallsonde, was zu einer verbesserten Bildqualität der Ultraschallaufnahmen führt.

Ferner wird die Aufgabe durch eine Personenaufnahme gelöst, die eine erfindungsgemäße Haltevorrichtung aufweist.

Die Haltevorrichtung kann somit Teil einer Personenaufnahme sein. Hierbei ist unter einer Personenaufnahme ein Bett, eine Liege, ein Operationstisch, eine Trage für Notfalleinsätze oder ähnliches zu verstehen. Hierbei kann eine Aussparung in der Personenaufnahme zur definierten Positionierung der Haltvorrichtung verwendet werden. Ferner ist eine in der Höhe verstellbare Haltevorrichtung möglich, indem beispielsweise die Haltevorrichtung vollständige oder zumindest teilweise in die Personenaufnahme bzw. deren Liegefläche integriert ist.

Die vorgeschlagene Haltevorrichtung kann zur Verwendung für eine Punktion vorgesehen werden, insbesondere für eine Punktion an einem Bein.

Mit der vorliegenden Erfindung wird eine Selbsthalterung für eine Ultraschallsonde bereitgestellt, die einem Arzt oder einer medizinischen Fachkraft das ultraschallgesteuerte Vorschieben einer Hohlnadel im Bereich rückseitiger Gewebeareale des menschlichen Körpers (wie beispielsweise Oberschenkel, Unterschenkel, Oberarm oder Unterarm) erleichtert. Bei der Verwendung einer Zielhilfe an der Haltevorrichtung kann eine Punktion besonders leicht durchgeführt werden. Insgesamt wird eine ultraschallgestützte Punktion ermöglicht.

Die Erfindung, sowie weitere Merkmale, Ziele, Vorteile und Anwendungsmöglichkeiten derselben wird bzw. werden nachfolgend anhand einer Beschreibung von bevorzugten Ausführungsbeispielen unter Bezugnahme auf die beigefügen Zeichnungen näher erläutert. In den Zeichnungen bezeichnen dieselben Bezugszeichen dieselben bzw. entsprechende Elemente. Dabei bilden alle beschriebenen und/oder bildlich dargestellten Merkmale für sich oder in beliebiger sinnvoller Kombination den Gegenstand der vorliegenden Erfindung, und zwar unabhängig von ihrer Zusammenfassung in den Patentansprüchen oder deren Rückbeziehung. Es zeigen:
Fig. 1 einen Anwendungsfall einer erfindungsgemäßen Haltevorrichtung während einer Verwendung auf einer Personenaufnahme und
Fig. 2 bis Fig. 19 Ausführungsbeispiele einer erfindungsgemäßen Haltevorrichtung.

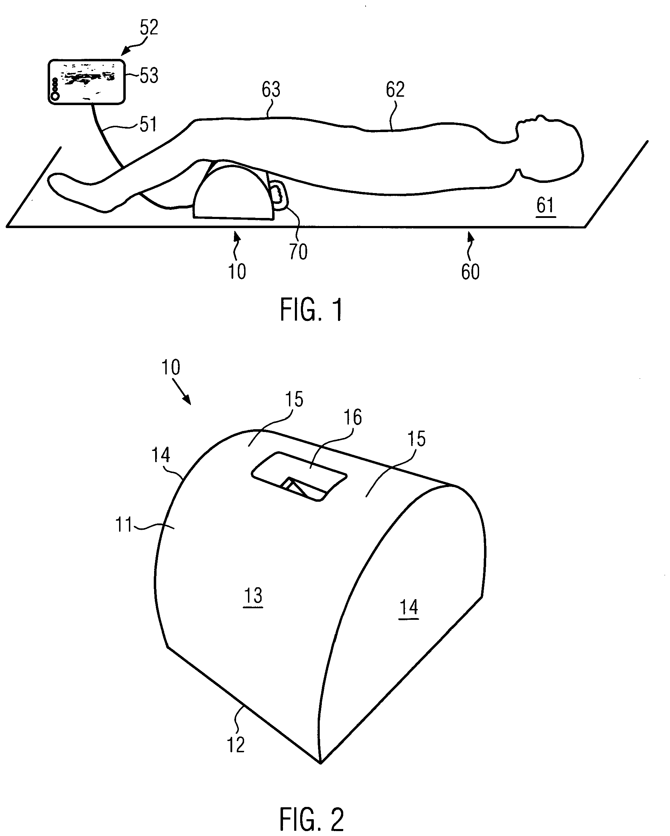

In den Fig. 1 bis 19 werden Ausführungsbeispiele gemäß der vorliegenden Erfindung dargestellt. Hierbei können die gezeigten und beschriebenen Merkmale unterschiedlicher Ausführungsbeispiele beliebig miteinander kombiniert werden.

Fig. 1 zeigt einen Anwendungsfall einer erfindungsgemäßen Haltevorrichtung 10 während einer Verwendung auf einer Personenaufnahme 60. Auf einer Liegefläche 61 der Personenaufnahme 60 liegt eine Person 62 bzw. ein Patient auf ihrem Rücken. Die Haltevorrichtung 10 ist auf der Liegefläche 61 im Bereich eines zu untersuchenden Körperteils 63, hier ein Oberschenkel der Person 62 positioniert, so dass die Rückseite des Oberschenkels 63 mit der Haltevorrichtung 10 in Kontakt kommt. Die Haltevorrichtung 10 weist einen Griff 70 auf. Mit Hilfe des Griffs 70 kann die Haltevorrichtung 10 auf der Liegefläche 61 verschoben werden, um eine geeignete Position der Haltevorrichtung 10 im Bereich des Oberschenkels 63 zu erreichen. In Fig. 1 ist der Griff 70 an einer Längsseite der Stützfläche 13 angeordnet, wobei die Stützfläche 13 beidseitig der erste Aufnahmeöffnung 16 in Richtung Grundfläche 12 verläuft. Die Haltevorrichtung 10 nimmt eine Ultraschallsonde 50 auf (in Fig. 1 durch den Oberschenkel 63 verdeckt), deren Kabel 51 an einem Ultraschallgerät 52 mit Anzeigeneinheit 53 angeschlossen ist. Die Haltevorrichtung 10 kann in dem gezeigten Anwendungsfall der Fig. 1 für eine Punktion des Beines einer Person 62 verwendet werden. Hierbei dient die Ultraschallsonde 50 beispielsweise für die Gewinnung von Echtzeitbildern während einer Punktion am Oberschenkel 63 der Person 62.

Fig. 2 zeigt ein Ausführungsbeispiel einer Haltevorrichtung 10, die einen einstückigen Grundkörper 11 aufweist. Der Grundkörper 11 weist eine Grundfläche 12, eine Stützfläche 13 und zwei Seitenflächen 14 auf. Die Stützfläche 13 dient zum Abstützen eines Körperteils der Person 62, so dass dieser abzustützende Körperteil in einem Auflagenbereich 15 der Stützfläche 13 aufliegt. Der Auflagebereich 15 bzw. die Auflagefläche 15 dient zur Stützung eines zu untersuchenden Körperteils, beispielsweise einen Oberschenkel 63 einer Person 62. Die Auflagefläche 15 befindet sich im bestimmungsgemäßen Gebrauch der Haltevorrichtung 10 in einem oberen Bereich des Grundkörpers 11.

Der Grundkörper 11 im Ausführungsbeispiel der Fig. 2 ist vorzugsweise symmetrisch ausgebildet, so dass die beiden Seitenflächen 14 bzw. Stirnflächen als identische Flächen ausgebildet sind, beispielsweise jeweils als eine halbkreisförmige oder parabelförmige Seitenfläche.

Ferner weist der Grundkörper 11 der Fig. 2 eine Aufnahmeöffnung 16 auf, um eine Ultraschallsonde 50 aufzunehmen. Die Aufnahmeöffnung 16 befindet sich im Bereich der Auflagefläche 15 und kann vorzugsweise mittig innerhalb der Auflagefläche 15 angeordnet sein. Auf diese Weise ist es möglich, dass das zu untersuchende Körperteil 63 von der Auflagefläche 15 gestützt wird und gleichzeitig eine Ultraschallsonde 50 innerhalb der Aufnahmeöffnung 16 aufgenommen ist, um das zu untersuchende Körperteil 63 mit Ultraschallwellen zu beschallen.

Die Ultraschallsonde 50 kann von der Auflagefläche 15 aus in den Grundkörper 11 eingeführt werden. Hierzu kann die Aufnahmeöffnung 16 der Fig. 2 so ausgestaltet sein, dass die Ultraschallsonde 50 zumindest teilweise im Inneren des Grundköpers 11 aufgenommen wird.

Durch das Auflegen des zu untersuchenden Körperteils 63 auf der Auflagefläche 15 kommt der Körperteil 63 mit der Ultraschallsonde 50 in Kontakt und ermöglicht durch das Eigengewicht des Körperteils 62 einen guten Kontakt zwischen der Ultraschallsonde 50 und dem zu untersuchenden Körperteil 63. Vorzugsweise schließt die obere Kante der Ultraschallsonde 50 mit der Auflagefläche 15 bündig ab, so dass die Auflagefläche 15 einschließlich der Ultraschallsonde 50 eine glatte Oberfläche bildet.

Die Ausführungsform der Fig. 2 kann insbesondere mit Vorteil für eine kabellose Ultraschallsonde 50 verwendet werden. Entsprechende Ausformungen im Inneren des Grundkörpers 11 können vorgesehen werden, um die Ultraschallsonde 50 vollständig in dem Grundköper 11 aufzunehmen. Der Grundköper 11 kann unterschiedliche Geometrien aufweisen. Seine Funktion ist es, die Ultraschallsonde 50 aufzunehmen und eine Auflagefläche 15 bereitzustellen. Der Grundkörper 11 hat in den dargestellten Figuren eine Grundfläche 12, eine Auflagefläche 15, eine Aufnahmeöffnung 16 und eine Geometrie zur Lagerung der aufzunehmenden Ultraschallsonde 50. In den Ausführungsformen der dargestellten Figuren sind Geometrien mit einer Seitenfläche 14 als bogen- oder parabelförmig Ausgestaltung vorgesehen. Die Seitenfläche 14 kann auch rechteckig, quadratisch, dreieckig, rund, elliptisch oder in einer sonstig geeigneten Form ausgebildet sein. Entsprechend weist in einem solchen Fall der Grundkörper 11 beispielsweise eine quaderförmige Form, eine keilförmige Form auf oder Kombinationen hieraus. Ein Gestell zur Gewichtsaufnahme des zu untersuchenden Körperteils 63 wäre hierzu auch möglich, das eine Ultraschallsonde 50 fixieren kann. Das Gestell kann hierbei eine Grundfläche aufspannen, um stabil positioniert zu werden. In einer weiteren Ausführungsform der Haltevorrichtung 10 kann beispielsweise ein beliebig geformter Schaumköper als Grundkörper 11 verwendet werden, um die Ultraschallsonde 50 aufzunehmen.

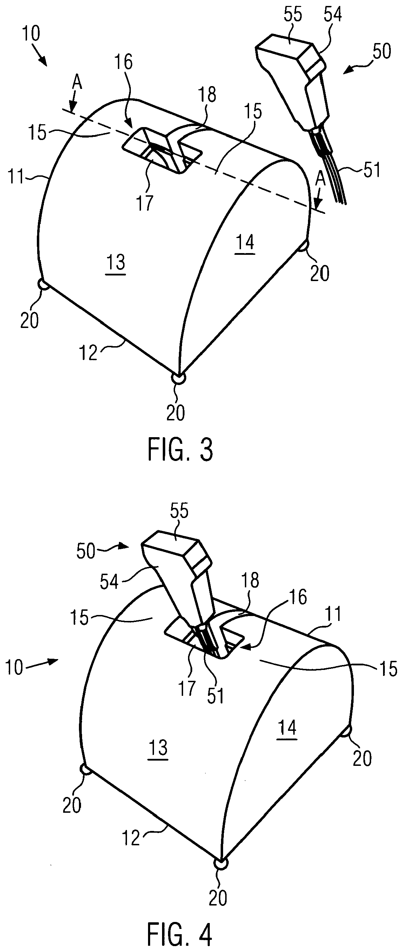

Bei einer Ultraschallsonde 50, die ein Kabel 51 aufweist, ist eine Ausnehmung 18 für das Kabel als Kabelführung von Vorteil, wie anhand der Fig. 3 und Fig. 4 näher erläutert wird. Die Kabelführung kann ein Kanal oder teilweise geöffneter Kanal sein und dient zur Aufnahme des Kabels 51 im Inneren des Grundkörpers 11. Ferner kann das Kabel 51 an der Grundfläche 12, der Stützfläche 13 oder einer Seitenflächen 14 aus dem Grundkörper 11 herausgeführt werden.

Ferner kann der Grundköper 11 der Fig. 2 elastisch ausgebildet sein, insbesondere kann der Grundkörper deformierbar sein, der sich deformiert, sobald ein zu untersuchender Körperteil 63 eines Patienten auf dem Grundkörper 11 gelagert wird. Dies hat den Vorteil, dass sich der Grundkörper 11 den Untersuchungsbedingungen anpassen kann, insbesondere kann sich der Grundkörper 11 aufgrund des Gewichtes des zu untersuchenden Körperteils verformen, so dass er sich eigenständig auf einer Liegefläche 61 als Untergrund fixiert, wie beispielsweise auf der Personenaufnahme 60 von Fig. 1. Ferner kann die Haltevorrichtung 10 Standfüße 20 aufweisen, die den Grundkörper 11 während eines bestimmungsgemäßen Gebrauches vor einem Verrutschen hindern. Solche Standfüße 20 sind beispielhaft in den Fig. 3 und 4 gezeigt.

Fig. 3 und Fig. 4 zeigen eine Ausführungsform einer Haltevorrichtung 10 zur Aufnahme einer Ultraschallsonde 50 mit einer Kabelführung. Fig. 3 zeigt die Ultraschallsonde 50 außerhalb der Haltevorrichtung 10, während Fig. 4 die gleiche Haltevorrichtung 10 während des Einführens der Ultraschallsonde 50 zeigt.

Die Ultraschallsonde 50 weist einen Schallkopf 54 und eine Schallfläche 55 auf. Ferner ist an der Ultraschallsonde 50 ein elektrisches Kabel 51 vorhanden, das die Ultraschallsonde 50 mit einer Auswerteeinheit bzw. deren Anzeigeeinheit 53 verbindet, um Aufnahmeinformationen zur Auswerteeinheit zu bringen. Es ist in den Fig. 3 und 4, ebenso wie bei dem Ausführungsbeispiel der Fig. 2 vorgesehen, dass die Ultraschallsonde 50 in dem Grundkörper 11 der Haltevorrichtung 10 vollständig aufgenommen werden kann. Aus der Schallfläche 52 der Ultraschallsonde 50 treten Ultraschallwellen aus, die durch Kontakt mit dem zu untersuchenden Körperteil 63 in das tiefere Gewebe des Körperteils 63 gelangen, um Ultraschallaufnahmen zu ermöglichen.

Der gezeigte Grundkörper 11 der Fig. 3 und Fig. 4 weist eine Aufnahmeöffnung 16 auf, die eine erste Ausnehmung 17 zur Aufnahme der Ultraschallsonde 50 und eine zweite Ausnehmung 18 zur Aufnahme zumindest einen Teil eines Kabels 51 der Ultraschallsonde 50 aufweist. Die erste Ausnehmung 17 und die zweite Ausnehmung 18 sind miteinander verbunden und bilden gemeinsam die Aufnahmeöffnung 16. Die erste Ausnehmung 17 erstreckt sich entlang einer Längsachse des Grundkörpers 11, während die zweite Ausnehmung 18 radial verläuft und einen Teil der Mantelfläche des Grundkörpers 11 bildet. Die zweite Ausnehmung 18 dient als Kabelführung bzw. als Kabelkanal. Der Kabelkanal kann an der Mantelfläche des Grundkörpers 11 offen sein, so dass man ohne weitere Maßnahmen das Kabel 51 von der Stützfläche 13 aus in das Innere des Grundköpers 11 einführen kann.

Vorteilhafterweise wird die Ultraschallsonde 50 zusammen mit dem Kabel 51 in den Grundkörper 11 eingebracht, falls beispielsweise diese beiden Teile der Ultraschallvorrichtung 52 fest miteinander verbunden sind.

Fig. 4 zeigt einen Zustand, in dem die Ultraschallsonde 50 nahezu eingeführt ist. Zunächst wird das Kabel 51 in die zweite Ausnehmung 18 eingeführt und danach wird der Schallkopf 54 in die erste Ausnehmung 17 geschoben, so dass seine Schallaustrittsfläche 55 bündig oder nahezu bündig mit der Fläche 15 abschließt.

Die Haltevorrichtung 10 der Fig. 3 und Fig. 4 weist ferner eine Vielzahl von Standfüßen 20 auf, die jeweils an der Mantelfläche des Grundkörpers 11 angeordnet sind. Die Standfüße 20 können steckbar ausgebildet sein, so dass ihre Positionen an der Mantelfläche des Grundkörpers 11 je nach Anwendungsfall variiert werden können. Im vorliegenden Ausführungsbeispiel sind die Standfüße 20 kugelförmig ausgebildet. Die Standfüße 20 können auch als Arretierungshilfe am Umfang des Grundkörpers 11 dienen. Typischerweise bilden sie eine Arretierungshilfe im Falle von zwei vorhandenen Standfüßen 13. Im Falle von drei oder vier vorhandenen Standfüßen 13, die eine Grundfläche aufspannen, werden die Standfüße 13 als Stützkörper verwendet, um eine Standfläche bzw. Grundfläche am Grundkörper 11 der Haltevorrichtung 10 zu definieren.

Die Standfüße 20 können rutschfest ausgebildet sein, beispielsweise aus Kunststoff oder gummiähnlichem Material, um die Haltevorrichtung 10 auch bei kleinerer Bewegung der Person 62 gegen Verrutschen der Haltevorrichtung 10 diese auf der Liegefläche 61 zu fixieren.

Hierbei begrenzen zwei oder vier Standfüße 20 in den Fig. 3 und Fig. 4 die Standfläche 12 im Bereich der Standfüße 20 des Grundkörpers 11. Zum Verhindern eines Verrutschens genügen bereits zwei Standfüße. Die Grundfläche 12 wird beispielsweise durch einen deformierbaren Teil des Grundkörpers 11 gebildet. Die Grundfläche 12 bildet sich im Bereich der Kontaktfläche des Grundkörpers 11 mit einem Untergrund, beispielsweise einer Patientenliege.

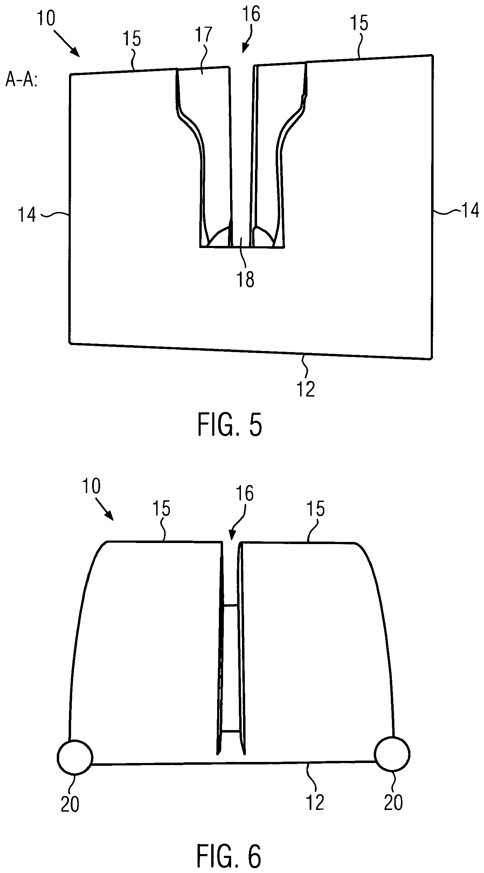

Fig. 5 zeigt einen Schnitt A-A durch den Grundkörper 11 der Fig. 3. Hierbei sind die Aufnahmeöffnung 16 sowie ein Teil der Kabelführung in Schnittansicht dargestellt. Die Aufnahmeöffnung 16 und ebenso der Grundkörper 11 weisen eine Mindesthöhe auf, die vorzugsweise mindestens der Höhe der Ultraschallsonde 50 entspricht. Dies bedeutet, dass die Ultraschallsonde 50 vollständig in dem Grundkörper 11 aufgenommen werden kann. Die Mindesthöhe liegt beispielsweise in einem Bereich von etwa 10 bis etwa 15 Zentimetern.

Die Kabelführung kann ferner einen Winkel aufweisen, so dass das Kabel 51 an einer Seitenfläche des Grundkörpers 11 in die Kabelführung eingeführt werden kann und gleichzeitig im bestimmungsgemäßen Gebrauch verstaut ist. Eine solche Ausgestaltung ist in Fig. 6 dargestellt.

Fig. 6 zeigt eine Rückansicht der Haltevorrichtung 10 mit einer Kabelführung, wie sie in Fig. 3 dargestellt ist. Der Grundkörper 11 der Haltevorrichtung 10 in Fig. 6 ist mit einer nach einer Seite vollständig geöffneten Kabelführung versehen, die an der Rückseite des Grundkörpers 11 verläuft.

Das Kabel 51 der Ultraschallsonde 50 kann somit abgewinkelt von der Haltevorrichtung 10 weggeführt werden. Der sich ergebene Winkel des geführten Kabels 51 kann aufgrund der Formgebung der Kabelführung beliebig ausgestaltet sein. Praktischerweise liegt der Winkel in einem Bereich von etwa 80 bis etwa 90 Grad, so dass das Kabel 51 auf einfache Weise nahezu in einem rechten Winkel von der Haltevorrichtung 10 aus der Kabelführung weggeführt werden kann. Dies hat den Vorteil, dass das Kabel 51 bei einer Untersuchung eines Patienten nicht weiter stört und vor Beschädigungen geschützt wird. Demnach kann die Kabelführung zumindest einen Kabelabschnitt des Kabels 51 aufnehmen.

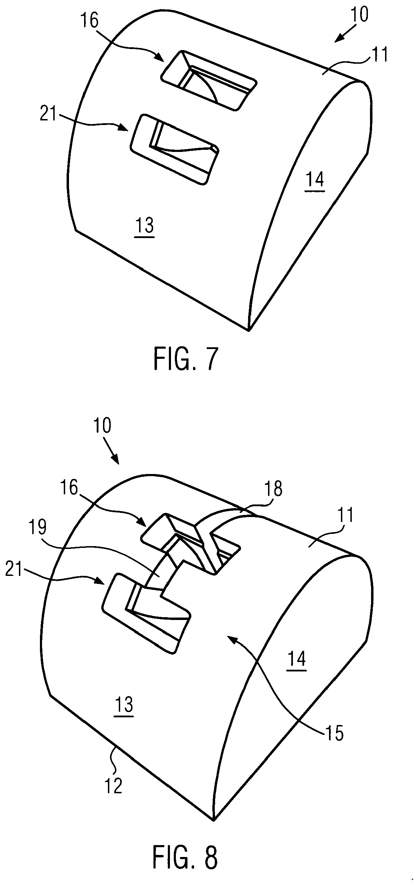

In den Fig. 7 bis 10 sind Ausführungsbeispiele einer Haltevorrichtung 10 gezeigt, die jeweils eine zweite Aufnahmeöffnung 21 aufweisen. Hierbei können die beschriebenen und gezeigten Merkmale einzeln oder in Kombination der Ausführungsbeispiele der Fig. 1 bis 6 mit den Ausführungsbeispielen der Fig. 7 bis 10 kombiniert werden.

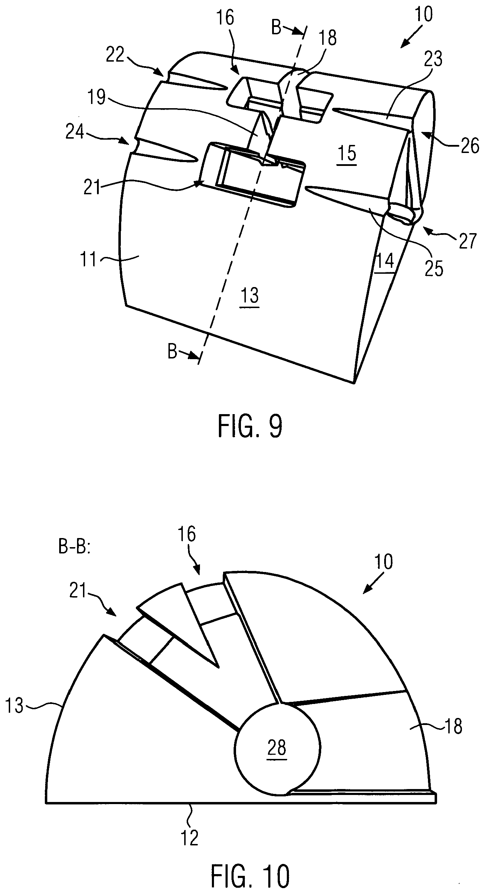

Fig. 8 und Fig. 9 zeigen jeweils ein Ausführungsbeispiel einer Haltevorrichtung 10 mit zwei Aufnahmeöffnungen 16, 21 am Grundkörper 11, die über eine Verbindungsöffnung 19 miteinander verbunden sind. In der Verbindungsöffnung kann beispielsweise ein Kabel 51 geführt werden.

Das Ausführungsbeispiel der Fig. 9 zeigt ferner eine Mehrzahl von Zielhilfen 22-25, die jeweils als Vertiefung ausgeführt sind. Jede der vier Zielhilfen 22-25 ist am Grundkörper 11 angeordnet und unabhängig voneinander zu verwenden. Die Vertiefungen der Zielhilfen 22-25 sind jeweils konisch ausgeführt und haben eine breitere Vertiefung am Rand des Grundkörpers 11, die sich in Richtung Aufnahmeöffnung 16, 21 verjüngt. Die Zielhilfen 22-25 enden auf der Auflagefläche 15 in der Nähe der Aufnahmeöffnungen 16, 21, ohne dass sie in diese münden. Das andere Ende der Vertiefungen der Zielhilfen 22-25 münden jeweils in die Seitenflächen 14 des Grundkörpers 11. Somit erstrecken sich die Zielhilfen 22-25 jeweils von der ersten bzw. zweiten Aufnahmeöffnung 16, 21 aus in Richtung Seitenwand 14 des Grundkörpers 11. Die Zielhilfen 22, 23 bzw. 24, 25 sind im vorliegenden Ausführungsbeispiel paarweise angeordnet. Es befinden sich jeweils zwei Zielhilfen seitlich von jeder der Aufnahmeöffnungen 16 bzw. 21. Die Zielhilfen 22-25 weisen eine konisch verlaufende Form auf, die eine geringere Abmessung an der Aufnahmeöffnung 16, 21 aufweist und eine größere Abmessung an der Seitenwand 14 des Grundkörpers 11 aufweist. Die Zielhilfen 22-25 dienen als Führung, für einen Arzt oder ärztliches Personal bei der Verwendung der Haltevorrichtung 10. Die Auflagefläche 15 wird durch die Zielhilfen 22-15 wenig beeinflusst, so dass ein Patient die Vertiefungen der Zielhilfen kaum spürt während der zu behandelnde Körperteil 63 hierauf liegt.

Ferner weist die Haltevorrichtung 10 der Fig. 9 Tasthilfen 26, 27 an der Seitenwand 14 auf. Die Tasthilfen 26, 27 springen von der Seitenwand 14 hervor und bilden jeweils einen Vorsprung. Ferner sind die Tasthilfen 26, 27 konisch ausgebildet und münden an einem Ende vorzugsweise in die Zielhilfen 22-25. Die Tasthilfen 26, 27 bilden jeweils eine Wölbung an der Seitenwand 14 aus. Somit bilden die Tasthilfen eine Positiv-Fläche, während die Ziehhilfen 22-15 eine Negativ-Fläche bilden. In Fig. 9 sind lediglich zwei Zielhilfen 26, 27 dargestellt, können jedoch auf der verdeckten Seitenfläche 14 der Fig. 9 ebenso vorhanden sein.

Fig. 10 zeigt eine Schnittansicht B-B in perspektivischer Darstellung einer Haltevorrichtung 10, wie sie beispielsweise in Fig. 9 dargestellt ist. Der Schnitt B-B liegt an der Seitenwand der Verbindungsöffnung 19 an. In der Schnittansicht B-B sind die Aufnahmeöffnungen 16, 21 mit einer dazwischenliegenden Verbindung 19 dargestellt. Ein Hohlraum 28 sorgt dafür, dass verschiedene geformte Ultraschallsonden 50 mit ihren unterschiedlich geformten Griffen darin gleichermaßen Platz finden.

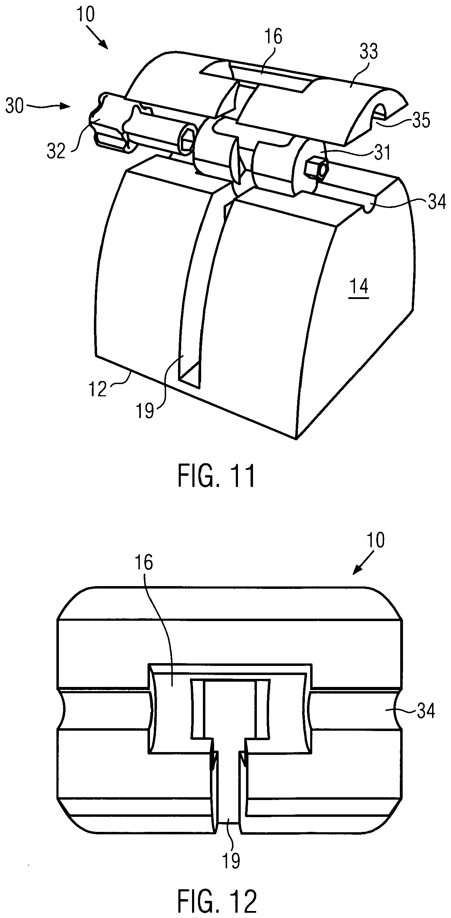

Fig. 11 zeigt eine weitere mögliche Ausführungsform einer erfindungsgemäßen Haltevorrichtung 10. Bei dieser Ausführungsform kann eine aufgenommene Ultraschallsonde 50 in dem Grundkörper 11 schwenkbar gelagert werden. Hierzu weist die Haltevorrichtung 10 einen Schwenkmechanismus in Form einer schwenkbaren Einrichtung 30 auf, der im oberen Bereich der Haltevorrichtung 10 oberhalb des Grundkörpers 11 aufgesetzt und befestigt ist. Der Schwenkmechanismus 30 weist eine Walze 31 auf, die drehbar an dem Grundkörper 11 gelagert ist. Zur Betätigung der Walze 31 ist ein Griff 32 vorhanden, der manuell oder automatisiert in Rotationsrichtung bewegt werden kann. Die Walze 31 kann mit einer Abdeckung 33 versehen werden. Die Walze 31 wie auch die Abdeckung 33 weisen eine Aufnahmeöffnung 16 auf, um die Ultraschallsonde 50 im bestimmungsgemäßen Gebrauch aufzunehmen. Der Grundkörper 11 weist eine Lagerungsführung 34 auf, die als halboffene Geometrie oder erste Teilöffnung ausgestaltet sein kann. Eine weitere Teilöffnung 35 ist vorzugsweise an Unterseite der Abdeckung 33 vorhanden, um einen Teilumfang der Walze aufnehmen zu können.

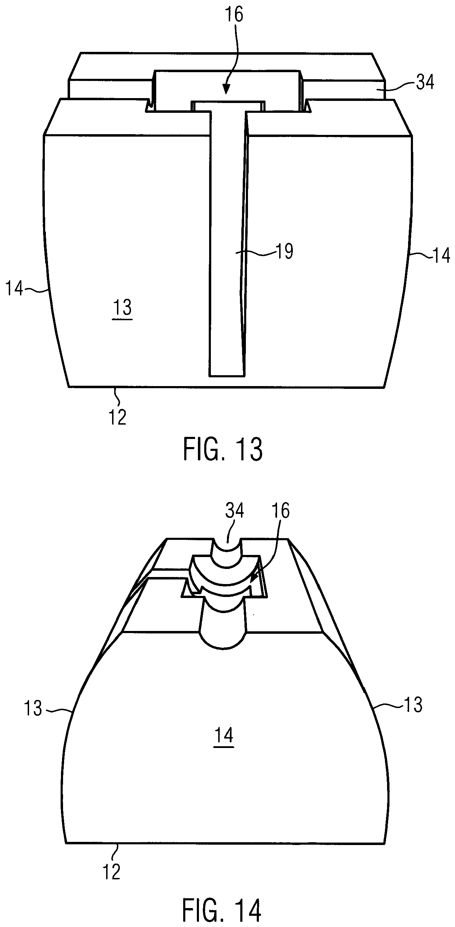

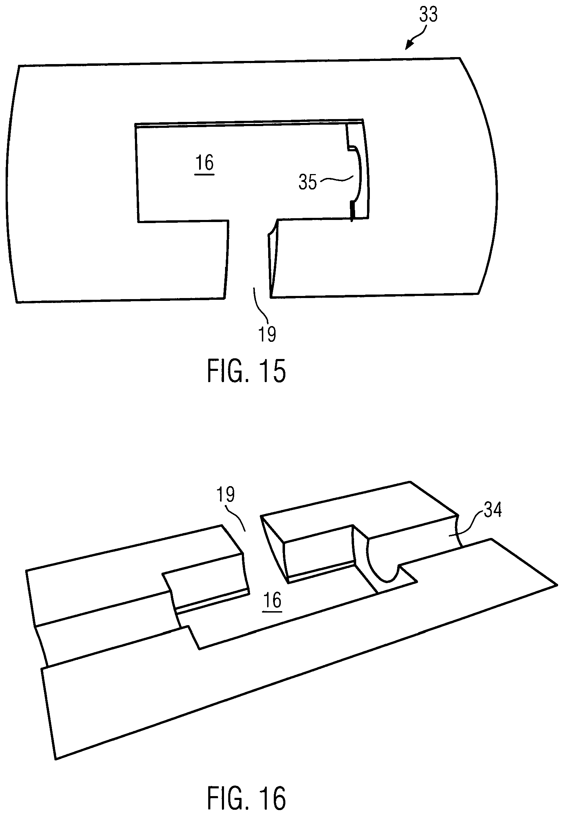

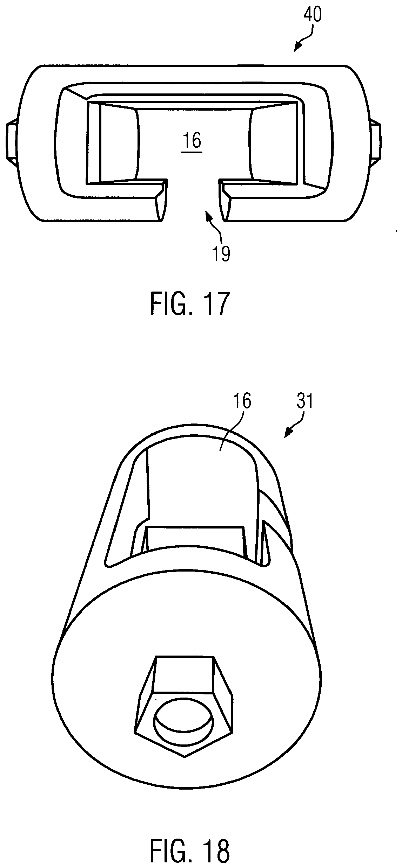

Die Figuren 12 bis 18 zeigen Detailansichten der in Fig. 11 dargestellten Haltevorrichtung 10. Hierbei zeigt Fig. 12 eine Draufsicht der Haltevorrichtung 10. Fig. 13 zeigt eine Rückansicht der Haltevorrichtung 10. Fig. 14 zeigt eine Seitenansicht der Haltevorrichtung 10. Fig. 15 zeigt eine Draufsicht der Abdeckung der Haltevorrichtung 10. Fig. 16 zeigt eine Unteransicht der Abdeckung der Fig. 15. Fig. 17 zeigt eine perspektivische Ansicht eines Ultraschalleinsatzes 40 mit einer Aufnahmeöffnung 16. Ferner zeigt Fig. 18 ein Ausführungsbeispiel einer Walze 31 einer Haltevorrichtung 10.

Der Ultraschalleinsatz 40 der Fig. 17 kann durch seine geometrische innere Formgebung unterschiedliche Ultraschallsonden 50 aufnehmen. Ein solcher Ultraschalleinsatz 40 kann beispielsweise auch in den Ausführungsbeispielen der Fig. 1 bis 10 verwendet werden. Auf diese Weise ist es möglich, eine einheitliche Öffnung bei der Produktion des Grundkörpers vorzusehen. Der Ultraschalleinsatz 40 dient als geometrischer Adapter, um unterschiedlich geformte Ultraschallköpfe mit deren unterschiedlichen Abmessungen in eine vorgefertigte Aufnahmeöffnung des Grundköpers einzusetzen. Die Aufnahmeöffnung 16 bzw. 21 kann somit in einer Produktion einheitlich erfolgen, ohne dass auf die unterschiedlichen Geometrien der später verwendeten Ultraschallköpfe Rücksicht genommen werden muss.

Die Walze 31 der in Fig. 11 gezeigten schwenkbaren Einrichtung 30 hat gleichzeitig die Funktionen einer Verlagerung und einer Halterung der Ultraschallsonde 50. Sie wird in eine entsprechend entgegengesetzt geformte Öffnung des Grundkörpers 11 eingefügt. Die Walze 31 kann beispielsweise mit Kugellager versehen sein, so dass wenig Kraft zur Verlagerung aufgewendet werden braucht. An beiden Seiten der Walze 31 kann ein beispielsweise als mehrkantiges Element, beispielsweise als Sechskant, oder einer anderen beliebigen Geometrie geformter Vorsprung angebracht sein, der es erlaubt eine Drehhilfe von außen an die Walze 31 anzubringen, welche dieselbe Geometrie als Vertiefung aufweist, um eine Drehbewegung während des Ultraschalluntersuchungsvorganges zu ermöglichen.

Der Grundkörper 11 der Haltevorrichtung 10 kann auf verschiedene Weisen hergestellt werden. Beispielsweise kann ein 3D-Druckverfahren oder ein Spritzgussverfahren zur Anwendung kommen. Bei solchen Herstellungsverfahren kann der Grundkörper 11 aus Kunststoff hergestellt werden. Auch kann der Grundkörper 11 im 3D-Druck aus Metall gefertigt sein. Ferner ist es auch möglich, den Grundkörper 11 aus einem Partikelschaum herzustellen. Hierbei sind expandiertes Polystyrol (EPS), expandiertes Polypropylen (EPP) oder expandiertes Polyethylen (EPE) besonders geeignet. Diese bestehen aus Schaumperlen mit Dichten im Bereich von etwa 15 bis etwa 80 kg/m³ und zeichnen sich durch sehr gute mechanische Eigenschaften, thermische Isolierfähigkeit und ein Leichtbaupotenzial aus. Die Verarbeitung der Schaumperlen zu Formteilen findet in speziellen Formteilautomaten mithilfe von Wasserdampf statt.

Die erfindungsgemäße Haltevorrichtung 10 und ihre Ausführungsbeispiele eignen sich besonders für die Vorbereitung und Durchführung einer Punktion, beispielsweise an einem Bein eines Patienten. Durch die Auflagefläche 15 der Haltevorrichtung 10 kann der Oberschenkel ruhig liegen während ein Arzt eine geeignete Einstichstelle für die Punktionsnadel anhand einer Ultraschallaufnahme auswählt und dann eine ultraschallgestützte Punktion durchführt.

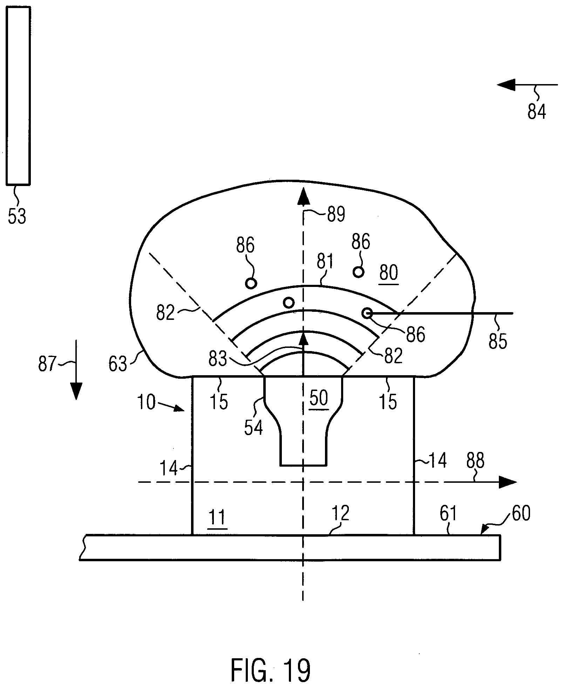

Fig. 19 zeigt schematisch einen Ausschnitt aus Fig. 1 mit einer Haltevorrichtung 10 für eine Ultraschallsonde 50 zu einer bildgebenden Darstellung eines zu untersuchenden Körperteils einer Person 62. Der zu untersuchende Körperteil 63 ist hierbei ein Oberschenkel der Person 62, wie in Fig. 1 dargestellt. Der Grundkörper 11 der Haltevorrichtung 10 weist eine Grundfläche 12 und eine Auflagefläche 15 auf. Hierbei ist die Auflagefläche 15 zur Stützung des zu untersuchenden linken Oberschenkels 63 ausgebildet ist. Der Oberschenkel 63 ist auf der Auflagefläche 15 des Grundkörpers 11 gelagert. Die Auflagefläche 15 ist ein oberer Bereich an dem Grundkörper 11, der mit dem Oberschenkel in Kontakt kommt und diesen stützt. Ferner weist der Grundköper 11 der Fig. 19 eine Aufnahmeöffnung 16 auf zum Aufnehmen einer Ultraschallsonde 50. Hierbei ist die Aufnahmeöffnung 16 in der Auflagefläche 15 derart angeordnet, so dass mit der aufgenommenen Ultraschallsonde 50 in der Aufnahmeöffnung 16 der zu untersuchende Körperteil aus der Auflagefläche 15 heraus beschallbar ist. Hierbei bilden die im Grundkörper 11 der Haltevorrichtung 10 aufgenommenen Ultraschallsonde 50 und die Auflagefläche 15 eine gemeinsame stufenfreie Fläche. Diese Fläche 15 ist gekrümmt bezüglich der aufgezeigten x-Achse 88 in Fig. 19, d.h. die x-Achse ist eine Rotationsachse der Auflagefläche 15. Ferner ist der Grundköper 11 bezüglich der aufgezeigten y-Achse 89 in Fig. 19 achsensymmetrisch, d.h. links und rechts der y-Achse 89 ist der Grundkörper 11 symmetrisch bezüglich seiner geometrischen Abmessungen sowie Aufnahmeöffnungen. Wie in einer perspektifischen Seitenansicht der Fig. 2 näher gezeigt, können die Seitenflächen 14 jeweils einen Ausschnitt aus einer rotationssymmetrischen Fläche oder einer Parabel bilden. Hierzu bildet die x-Achse 88 der Fig. 19 eine beispielhafte Rotationsachse.

Für die gezeigte Untersuchung an einem Patienten in Fig. 1 und Fig. 19 wird die Haltevorrichtung 10 unter dem zu untersuchenden Oberschenkel 63 der auf dem Rücken liegenden Person auf der Personenliege mit Liegefläche 61 platziert. Dies kann durch leichtes Anheben des Beins der Person geschehen, ohne dass die Person aus ihrer Rückenlage verlagert werden muss. Die Haltevorrichtung 10 kann auf der Liegefläche 61 im Anschluss nach ihrer Platzierung unter dem Oberschenkel arretiert werden, beispielsweise durch eine Steckverbindung, Saugverbindung, einer Klickverbindung oder ähnlichem.

Zur Untersuchung werden Schallwellen der Ultraschallsonde 50 technisch genutzt, um im vorliegenden Fall der Fig. 19 eine Nervenbahn 86 mit einem Narkotikum zu punktieren. Die anvisierte Nervenbahn 86 ist von Muskelgewebe und Sehnenstrukturen umschlossen. Ferner sind weitere Nerven- und Blutbahnen im untersuchten Körperareal, das als Querschnitt in Fig. 19 innerhalb des Oberschenkels 63 schematisch gezeigt ist.

Für die Punktion wird eine Punktionsnadel 85 mit einer Nadelspitze in den Oberschenkel 63 eingeführt, um an die Nervenbahn 86 ein Narkotikum abzugeben. Das Narkotikum bewirkt eine örtliche Betäubung. Die Punktion kann eine Vorbereitung für eine medizinische Untersuchung oder eine Operation sein.

In Fig. 19 weist die Ultraschallsonde 50 einen schematisch gezeigten Schallbereich 80 mit einem Öffnungswinkel 82 auf, in dem sich Ultraschallwellen 81 räumlich ausbreiten. In einer zweidimensionalen Ansicht ist dies mit der Schallausbreitungsrichtung 83 in Fig. 19 dargestellt. Mit dem Pfeil 84 ist eine Blickrichtung eines Arztes angezeigt, der während der Untersuchung des Patienten 62 seitlich neben der Personenaufnahme 60 bzw. der Patientenliege steht oder sitzt. Die Blickrichtung 84 des Arztes ist im bestimmungsgemäßen Gebrauch der Haltevorrichtung 10 orthogonal, d.h. rechtwinklig oder annähernd orthogonal zu der Schallausbreitungsrichtung 83 der Ultraschallsonde 50. Auf diese Weise ist es möglich, dass der Arzt die Ausbreitung der Ultraschallwellen 81 durch das Einführen der Punktionsnadel 85 in das Bein 63 selbst kaum beeinflusst. Es ist von Vorteil, dass der Blickrichtung 84 des Arztes orthogonal oder annähernd orthogonal zur Ausbreitungsrichtung 83 und Aufnahmerichtung der Ultraschallsonde 50 ist. Daher braucht der Arzt die Punktionsnadel 85 nur nahezu senkrecht in Richtung des auf einer Anzeigeeinheit 53 eines Ultraschallgerätes 52 dargestellten Anästhesiebereiches einstechen, um sicher zu sein, dass er zielsicher in dieser Ebene den anvisierten Bereich mit der Nervenbahn 86 trifft.

Die Beschallungsausrichtung 81 der Ultraschallsonde 50 und die Blickrichtung 84 bzw. die Blickbereich 85 des Arztes sind so aufeinander abgestimmt, dass die Spitze der Punktionsnadel 85 im Ultraschallbild gut sichtbar bleibt. Wenn sich die Schallausbreitungsrichtung 83 stark von der Blickrichtung 84 des Arztes unterscheidet entstehen Schwierigkeiten, um einen bestimmten Punkt im Körperareal zu treffen. Daraus ergibt sich, dass der Arzt beim Einstechen der Nadel nicht oder nur sehr ungenau weiß, wo diese hinführt und kann diese aber mangels konkreter Anhaltspunkte nicht richtig koordinieren. Dies wird im vorliegenden Fall aufgrund der Anordnung vermieden.

In Fig. 19 wird die Schallkopfpositionierung relativ zum Oberschenkel in der kurzen Achse ausgerichtet (Querschnitt des Oberschenkels), um ein oder mehrere zweidimensionale oder mehrdimensionale Darstellungen von Schnittebenen des Oberschenkels aufzunehmen. Es können Bildinformationen in Echtzeit produziert werden. Mit der vorgeschlagenen Haltevorrichtung ist somit im medizinischem Sinne ein ventraler Transversalschnitt als Ultraschall-Aufnahme möglich, d.h. der Schallkopf wird quer zum Oberschenkel positioniert (transversal), um von der Rückseite her (dorsal) den Oberschenkel zu beschallen.

In Fig. 19 ist die Ausstrahlung der Schallwellen 81 aus der Ultraschallsonde 50 entgegen der Schwerkraftrichtung 87 gerichtet. Auf diese Weise kann der Kontakt zwischen Schallkopf 54 und Köperteil 63 verbessert werden, da durch die Schwerkraftwirkung das zu untersuchende Körperteil 63 in Richtung Auflagefläche 15 bzw. Schallkopf 54 drückt. In dem mittigen Bereich der Auflagefläche 15 treten Schallwellen 83 entgegen der Schwerkraft 87 aus dem Schallkopf 54 aus.

Insgesamt wird gemäß der Erfindung und ihren Ausführungsbeispielen eine Haltevorrichtung 10 für eine Ultraschallsonde 50 zur bildgebenden Darstellung, vorzugsweise in Echtzeit-Darstellung, von zu untersuchenden Körperteilen 63 einer zu untersuchenden bzw. zu therapierenden Person vorgesehen. Hierbei wird die Haltevorrichtung 10 so ausgestaltet werden, dass diese auf einer Personenaufnahme für eine darauf zu platzierende Person bzw. Extremität der Person verwendet werden kann. Ferner ist in einem oberen Bereich der Haltevorrichtung 10 mindestens eine Aufnahmeöffnung 16 vorgesehen werden, in die eine Ultraschallsonde 50 einbringbar ist mit Schallausrichtung bzw. Schallausbreitung 81 zum Körper der zu untersuchenden Person.

Es ergeben sich zahlreiche Vorteile aus der vorgeschlagenen Erfindung und deren Ausführungsbeispiele, von denen hier einige genannt werden.

Es ist von Vorteil, dass die Ultraschallsonde vollständig in den Grundkörper der Haltevorrichtung aufgenommen wird. Eine solche Integration ist platzsparend und schützt die Ultraschallsonde während des Betriebs. Gleichzeitig dient der Grundköper als Halterung und Fixierung der Ultraschallsonde. Auf diese Weise kann der Arzt seine beiden Hände für die eigentliche Untersuchung verwenden, ohne dass er sich um die Fixierung und Positionierung der Ultraschallsonde kümmern muss. Dies verbessert die Qualität der Untersuchung.

Ein Vorteil der vorgeschlagenen Haltevorrichtung und deren Ausführungsformen ist, dass die Aufnahmerichtung der Ultraschallsonde der Visierrichtung des Arztes bzw. der Behandlungsperson entspricht. Auf diese Weise kann eine gezielte Positionierung einer Punktionsnadel sehr präzise ermöglicht werden.

Ferner ist von Vorteil, wenn keine weiteren Vorrichtungen, wie beispielsweise bisher übliche Aufnahmearme für eine Ultraschallsonde im Behandlungsbereich des Arztes angeordnet sind. Durch die Geometrie der Haltevorrichtung wird die Bewegungsfreiheit des Arztes nicht eingeschränkt, da keine weiteren Haltevorrichtungen für die Ultraschallsonde notwendig sind. Ferner ist von Vorteil, dass die vorgeschlagene Haltevorrichtung Zeit für die Vorbereitung der Untersuchung einspart. Ein Grund hierfür ist, dass bei einer Untersuchung einer Körperrückseite eines Patienten eine Seitenlage bzw. eine Verlagerung des Patienten notwendig ist. Eine solche Verlagerung führt oft zu Schmerzen, die vermieden werden können, da keine Umlagerung notwendig ist. Mit der vorgeschlagenen Haltevorrichtung kann der Patient in seiner Rückenlage unverändert bleiben.

**Bezugszeichenliste**

| | | | |
|---|---|---|---|
| 10 | Haltevorrichtung | 40 | Ultraschalleinsatz |
| 11 | Grundkörper | 50 | Ultraschallsonde |
| 12 | Grundfläche | 51 | Kabel |
| 13 | Stützfläche | 52 | Ultraschallgerät |
| 14 | Seitenflächen | 53 | Anzeigeneinheit |
| 15 | Auflagefläche | 54 | Schallkopf |
| 16 | erste Aufnahmeöffnung | 55 | Schallfläche |
| 17 | erste Ausnehmung | 60 | Personenaufnahme |
| 18 | zweite Ausnehmung | 61 | Liegefläche |
| 19 | Verbindungsöffnung | 62 | Person |
| 20 | Standfuß | 63 | zu untersuchender Körperteil |
| 21 | zweite Aufnahmeöffnung | 70 | Griff |
| 22-25 | Zielhilfe | 80 | Schallbereich |
| 26 | Tasthilfe | 81 | Ultraschallwellen |
| 27 | Tasthilfe | 82 | Öffnungswinkel |
| 28 | Hohlraum | 83 | Schallausbreitung |
| 30 | schwenkbare Einrichtung | 84 | Blickrichtung |
| 31 | Walze | 85 | Punktionsnadel |
| 32 | Drehgriff | 86 | Nervenbahn |
| 33 | Abdeckung | 87 | Schwerkraftrichtung bzw. Gravitationsrichtung |
| 34 | Lagerungsführung | | |
| 35 | Teilöffnung der Lagerungsführung | 88 | x-Achse |
| | | 89 | y-Achse |

## Patentansprüche

1. Haltevorrichtung (10) für eine Ultraschallsonde (50) zu einer bildgebenden Darstellung eines zu untersuchenden Körperteils (63) einer Person (62) aufweisend
einen Grundkörper (11) mit einer Grundfläche (12), wobei der Grundkörper (11) eine Aufnahmeöffnung (16) aufweist zur Aufnahme einer Ultraschallsonde (50),
wobei eine Auflagefläche (15) zur Stützung des zu untersuchenden Körperteils (63) ausgebildet ist, und wobei die Aufnahmeöffnung (16) in der Auflagefläche (15) angeordnet ist und mit einer aufgenommenen Ultraschallsonde (50) in der Aufnahmeöffnung (16) der zu untersuchende Körperteil (63) aus der Auflagefläche (15) heraus beschallbar ist,
**dadurch gekennzeichnet, dass**
der zu untersuchende Körperteil (63) ein Oberschenkel einer Person (62) ist.

2. Haltevorrichtung (10) nach Anspruch 1, wobei der Grundkörper (11) einstückig ausgebildet ist.

3. Haltevorrichtung (10) nach Anspruch 1 oder Anspruch 2, wobei der Grundkörper (11) deformierbar ist.

4. Haltevorrichtung (10) nach mindestens einem der Ansprüche 1 bis 3, ferner aufweisend eine Kabelführung.

5. Haltevorrichtung (10) nach mindestens einem der Ansprüche 1 bis 4, ferner aufweisend eine Zielhilfe (22-25).

6. Haltevorrichtung (10) nach mindestens einem der Ansprüche 1 bis 5, ferner aufweisend eine Tasthilfe (26, 27).

7. Haltevorrichtung nach mindestens einem der Ansprüche 1 bis 6, ferner aufweisend eine schwenkbare Einrichtung (30) zur Aufnahme und Schwenkung der Ultraschallsonde (50).

8. Haltevorrichtung (10) nach mindestens einem der Ansprüche 1 bis 7, wobei die Haltevorrichtung (10) einen Griff (70) aufweist.

9. Haltevorrichtung (10) nach mindestens einem der Ansprüche 1 bis 8, wobei die im Grundkörper aufgenommene Ultraschallsonde (50) und die Auflagefläche (15) eine gemeinsame stufenfreie Fläche bilden.

10. Haltevorrichtung (10) nach mindestens einem der Ansprüche 1 bis 9, wobei der Grundkörper (11) eine rechteckige Grundfläche (12) aufweist.

11. Haltevorrichtung (10) nach mindestens einem der Ansprüche 1 bis 10, wobei die Ausstrahlung von Schallwellen aus der Ultraschallsonde (50) entgegen der Schwerkraftrichtung (87) gerichtet ist.

12. Haltevorrichtung (10) nach mindestens einem der Ansprüche 1 bis 11, wobei der Grundköper (11) achsensymmetrisch ist.

13. Haltevorrichtung (10) nach mindestens einem der Ansprüche 1 bis 12, wobei im bestimmungsgemäßen Gebrauch der Haltevorrichtung (10) mit Hilfe der Schwerkraft der zu untersuchende Körperteil (63) auf die Auflagefläche (15) gedrückt wird.

14. Personenaufnahme (60) aufweisend eine Haltevorrichtung (10) nach mindestens einem der Ansprüche 1 bis 13.

## Claims

1. Holding apparatus (10) for an ultrasonic probe (50) for image-based representation of a body part (63) to be examined of a person (62) comprising
a base body (11) having a base area (12), wherein the base body (11) comprises a receiving opening (16) for receiving an ultrasonic probe (50),
wherein a bearing surface (15) is formed to support the body part (63) to be examined, and wherein the receiving opening (16) is arranged in the bearing surface (15) and the body part to be examined is treatable with sound with a received ultrasonic probe (50) in the receiving opening (16) out of the bearing surface (15),
**characterized in that**,
the body part (63) to be examined is a thigh of a person (62).

2. Holding apparatus (10) according to claim 1, wherein the base body (11) is integrally formed.

3. Holding apparatus (10) according to claim 1 or claim 2, wherein the base body (11) is deformable.

4. Holding apparatus (10) according to at least one of claims 1 to 3, further comprising a cable guide.

5. Holding apparatus (10) according to at least one of claims 1 to 4, further comprising an aiming aid (22-25).

6. Holding apparatus (10) according to at least one of claims 1 to 5, further comprising a tactile aid (26, 27).

7. Holding apparatus according to at least one of claims 1 to 6, further comprising a pivotable device (30) for receiving and pivoting the ultrasonic probe (50).

8. Holding apparatus (10) according to at least one of claims 1 to 7, wherein the holding apparatus (10) comprises a handle (70).

9. Holding apparatus (10) according to at least one of claims 1 to 8, wherein the ultrasonic probe (50) received in the base body and the bearing surface (15) form a common step-free area.

10. Holding apparatus (10) according to at least one of claims 1 to 9, wherein the base body (11) comprises a rectangular base area (12).

11. Holding apparatus (10) according to at least one of claims 1 to 10, wherein the emission of sound waves from the ultrasonic probe (50) is directed against the gravity direction (87).

12. Holding apparatus (10) according to at least one of claims 1 to 11, wherein the base body (11) is axially symmetric.

13. Holding apparatus (10) according to at least one of claims 1 to 12, wherein in the intended use of the holding apparatus (10) the body part (63) to be examined is pressed onto the bearing surface (15) with the aid of gravity.

14. Receiving device for a person (60) comprising a holding apparatus (10) according to at least one of claims 1 to 13.

## Revendications

1. Dispositif de maintien (10) pour une sonde à ultrasons (50) pour une représentation par imagerie d'une partie de corps à examiner (63) d'une personne (62), présentant un corps de base (11) comprenant une surface de base (12), dans lequel le corps de base (11) présente une ouverture de réception (16) pour recevoir une sonde à ultrasons (50),
dans lequel une surface d'appui (15) est formée pour supporter la partie de corps à examiner (63), et dans lequel l'ouverture de réception (16) est disposée dans la surface d'appui (15), et avec une sonde à ultrasons (50) reçue dans l'ouverture de réception (16), la partie de corps à examiner (63) peut être soumise à des ultrasons depuis la surface d'appui (15),
**caractérisé en ce que**
la partie de corps à examiner (63) est une cuisse d'une personne (62).

2. Dispositif de maintien (10) selon la revendication 1, dans lequel le corps de base (11) est formé d'un seul tenant.

3. Dispositif de maintien (10) selon la revendication 1 ou la revendication 2, dans lequel le corps de base (11) est déformable.

4. Dispositif de maintien (10) selon l'une au moins des revendications 1 à 3 présentant en outre un guide de câble.

5. Dispositif de maintien (10) selon l'une au moins des revendications 1 à 4 présentant en outre une assistance de visée (22-25).

6. Dispositif de maintien (10) selon l'une au moins des revendications 1 à 5 présentant en outre une assistance tactile (26, 27).

7. Dispositif de maintien (10) selon l'une au moins des revendications 1 à 6 présentant en outre un dispositif pivotable (30) pour la réception et le pivotement de la sonde à ultrasons (50).

8. Dispositif de maintien (10) selon l'une au moins des revendications 1 à 7, dans lequel le dispositif de maintien (10) comporte une poignée (70).

9. Dispositif de maintien (10) selon l'une au moins des revendications 1 à 8, dans lequel la sonde à ultrasons (50) reçue dans le corps de base et la surface d'appui (15) forment une surface commune sans décrochement.

10. Dispositif de maintien (10) selon l'une au moins des revendications 1 à 9, dans lequel le corps de base (11) présente une surface de base rectangulaire (12).

11. Dispositif de maintien (10) selon l'une au moins des revendications 1 à 10, dans lequel le rayonnement d'ondes sonores à partir de la sonde à ultrasons (50) est dirigé à l'opposé de la direction de la gravité (87).

12. Dispositif de maintien (10) selon l'une au moins des revendications 1 à 11, dans lequel le corps de base (11) est axisymétrique.

13. Dispositif de maintien (10) selon l'une au moins des revendications 1 à 12, dans lequel lors d'un usage conforme du dispositif de maintien (10), la partie de corps à examiner (63) est pressée sur la surface d'appui (15) par gravité.

14. Élément de réception de personne (60) présentant un dispositif de maintien (10) selon l'une au moins des revendications 1 à 13.
